# EUROPEAN PATENT SPECIFICATION

(11) **EP 3 111 901 B1**
(45) Date of publication and mention of the grant of the patent: **26.12.2018**
(21) Application number: 15755824.8
(22) Date of filing: 08.01.2015
(51) Int. Cl.: A61F 13/15, A61F 13/49, A61F 13/496

(54) **METHOD FOR MANUFACTURING ABSORBENT ARTICLE**
VERFAHREN ZUR HERSTELLUNG EINES SAUGFÄHIGEN ARTIKELS
PROCÉDÉ DE FABRICATION D'ARTICLE ABSORBANT

(30) Priority: 28.02.2014 JP 2014038859
(43) Date of publication of application: 04.01.2017
(73) Proprietor: Unicharm Corporation, Ehime 799-0111 (JP)
(72) Inventor: YAMAMOTO, Hiroki, Kanonji-shi Kagawa 769-1602 (JP); MATSUMOTO, Yoshihiko, Kanonji-shi Kagawa 769-1602 (JP)
(74) Representative: Dolleymores
(86) International application number: PCT/JP2015/050337
(87) International publication number: WO 2015/129297

(56) References cited:
- WO-A1-2013/073410
- WO-A1-2013/073411
- WO-A1-2013/094591
- JP-A- H07 213 553
- JP-A- 2000 070 300
- JP-A- 2000 070 300
- JP-A- 2008 161 525
- JP-A- 2009 189 621
- JP-A- 2013 202 052
- JP-B1- 5 360 327

## Description

### Technical Field

The invention relates to a method for manufacturing an absorbent article.

### Background Art

In a manufacturing line for absorbent articles such as a disposable diaper, the following method is commonly used for manufacturing pull-on diapers: the end sections of the continuous sheet in the transverse direction are folded to be layered on a ventral part and on a dorsal part while the continuous sheet is transported in the transport direction, the continuous sheet being to be an exterior material of a disposable diaper, the transport direction being a direction in which the continuous sheet continues; then, both end sections of the continuous sheet are each joined; and thereafter a single-cut piece is cut and separates from the continuous sheet. As methods for joining the transverse end sections of the ventral part of a diaper to the transverse end sections of the dorsal part, methods by welding such as ultrasonic sealing or heat sealing are widely known. For example, in Patent Literature 1, a plurality of band-like sheets which constitute an exterior material are joined with adhesive while extended elastic members are sandwiched between the band-like sheets. And, the ventral part and the dorsal part are joined by welding the sheets on the adhesive-applied area, and as a result, a diaper is formed. In addition, elastic force of elastic members is provided to a diaper, and this allows a user to feel good body fit.

### Citation List

### [Patent Literature]

[Patent Literature 1] Japanese Patent Application Laid-open Publication No. 2013-126528
WO 2013/073410 A1, JP H07 213553 A and JP 5360327 B1 all relate to forming side seal portions for pant-type disposable diapers using adhesive and/or heat seals.

### Summary of the Invention

### Technical Problem

In some cases, welding on an adhesive-applied area of the sheets, as in Patent Literature 1, causes some problems. For example, due to the oil contained in adhesive, there is a possibility that welding strength of ultrasonic sealing is not sufficient and that adhesive which has put on a welding device stops continuous production of diapers. These troubles can be prevented if adhesive is not applied to areas to be welded. But, in this case, elastic members do not adhere to the exterior material of a diaper on the areas to which adhesive is not applied. Accordingly, on the areas, stretching-and-contraction force is not exerted. When a diaper in such a state is cut and separated from the continuous sheet, the elastic members are cut on both transverse end sections of the diaper, and the elastic members contract from both transverse end sections towards the transverse center of the diaper. On the other hand, stretching-and-contraction force due to elastic members is not exerted on areas of both transverse end sections on which adhesive is not applied, and therefore the exterior material does not contract. That is, only the elastic members contract, but the exterior material does not contract. Accordingly, the exterior material looks extending transversely outwardly beyond both transverse end sections, and this may deteriorate the appearance of a diaper.

In view of the problem described above, it is an advantage of the invention to provide a pull-on diaper in which the welding strength of the side parts thereof is sufficient and whose appearance is good.

### Solution to Problem

The present invention provides the method for continuously manufacturing an absorbent article of independent claim 1. The dependent claims specify preferred but optional features.

An aspect of the invention to achieve the above advantage is a method for continuously manufacturing an absorbent article,
the absorbent article being a pull-on absorbent article
that has a longitudinal direction and a transverse direction intersecting the longitudinal direction, and
that includes
a lower exterior member located on a side towards a wearer's garment,
an upper exterior member stacked on the lower exterior member from a side towards a wearer's skin,
an absorbent main body stacked on the upper exterior member from the side towards a wearer's skin, for absorbing excrement,
the method, including:
while a lower bandlike member and an upper bandlike member are transported in a transport direction that is along the transverse direction,
   the lower bandlike member including the lower exterior members that continue in a band-like manner in the transverse direction,
   the upper bandlike member including the upper exterior members that continue in a band-like manner in the transverse direction,
a process in which, a cutting position is determined, and
   an adhesion area is formed by putting adhesive on a first area of at least either one of the lower bandlike member and the upper bandlike member,
   the cutting position being to serve as end sections of the lower exterior member and the upper exterior member in the transverse direction when the lower exterior member and the upper exterior member are cut and separated from the lower bandlike member and the upper bandlike member,
   the first area including the cutting position and having a predetermined width in the transport direction;
a process in which
   a plurality of elastic members are placed so that a part of each of the elastic members overlaps the adhesion area,
   the elastic members being stretched in the transport direction;
a process in which
   the lower bandlike member and the upper bandlike member are stacked such that the elastic members are sandwiched between the lower and upper bandlike members, and
   the upper bandlike member adheres to the lower bandlike member and the elastic members on the adhesion area;
a process in which
   the lower bandlike member and the upper bandlike member are folded on a center portion in an intersecting direction so that the upper bandlike member is located inside the lower bandlike member,
   the intersecting direction intersecting the transport direction;
a process in which
   a welded section on which the folded lower bandlike member and the folded upper bandlike member are to be welded to each other is formed on a second area,
   the second area being located on both sides of the first area in the transport direction and having a predetermined width in the transport direction; and
a process in which
   the lower bandlike member, the upper bandlike member and the elastic members are cut together on the cutting position, and
   the lower exterior member and the upper exterior member which are shaped in the form of underpants are cut and separated from the lower bandlike member and the upper bandlike member;
wherein the second area does not overlap the first area within at least a partial area along the transport direction,
the second area is defined so that a distance from the cutting position to an end section of the second area in the transport direction that is closer to the cutting position is smaller than a distance from the cutting position to an end section of the first area in the transport direction.

Other features of this invention will become apparent from the description in this specification and the attached drawings.

### Effects of the Invention

According to the invention, it is possible to provide a pull-on diaper in which the welding strength of the side parts thereof is sufficient and whose appearance is good.

### BRIEF DESCRIPTION OF THE DRAWINGS

FIG. 1A is a plan view of a disposable diaper 1 which is spread out.
FIG. 1B is a cross-sectional view taken along line B-B in FIG. 1A.
FIG. 2 is a schematic perspective view of a diaper 1 which is shaped in the form of underpants.
FIG. 3 is a flow chart of processes for manufacturing diapers 1 of the first embodiment.
FIG. 4 is a diagram illustrating how the substrate of the diapers 1 is transported in the transport direction.
FIG. 5 is a diagram illustrating a first area and an adhesion area.
FIG. 6 is a diagram illustrating how the folded substrate of diapers 1 is transported after the process of S105.
FIG. 7 is a schematic side view of a welding-and-cutting apparatus 50 used in the first embodiment.
FIG. 8 is a diagram illustrating a heating block 521 of the welding-and-cutting apparatus 50.
FIG. 9 is a diagram illustrating areas where welded section is formed in welding process.
FIG. 10 is a magnified view of the area B in FIG. 9 and illustrates second areas and welded sections.
FIG. 11 is a diagram showing an example of a case in which adhesive is applied to an area except for the first area and the second area.
FIG. 12 is a schematic diagram of D-D section in FIG. 10.
FIG. 13 is a diagram illustrating how a bandlike member and an elastic member are cut in cutting process.
FIG. 14 is a plan view of a disposable diaper 2 which is spread out.
FIG. 15 is a flow chart of processes for manufacturing the diapers 2 in the second embodiment.
FIG. 16 is a diagram illustrating how the substrate of the diapers 2 is transported in the transport direction.

### Mode for Carrying Out the Invention

At least the following matters will be made clear by the description in the present specification and the accompanying drawings.

A method for continuously manufacturing an absorbent article,
the absorbent article being a pull-on absorbent article
that has a longitudinal direction and a transverse direction intersecting the longitudinal direction, and
that includes
a lower exterior member located on a side towards a wearer's garment,
an upper exterior member stacked on the lower exterior member from a side towards a wearer's skin,
an absorbent main body stacked on the upper exterior member from the side towards a wearer's skin, for absorbing excrement,
the method, including:
while a lower bandlike member and an upper bandlike member are transported in a transport direction that is along the transverse direction,
   the lower bandlike member including the lower exterior members that continue in a band-like manner in the transverse direction,
   the upper bandlike member including the upper exterior members that continue in a band-like manner in the transverse direction,
a process in which, when the lower exterior member and the upper exterior member are cut and separated from the lower bandlike member and the upper bandlike member,
   a cutting position is determined, and
   an adhesion area is formed by putting adhesive on a first area of at least either one of the lower bandlike member and the upper bandlike member,
      the cutting position being to serve as end sections of the lower exterior member and the upper exterior member in the transverse direction,
      the first area including the cutting position and having a predetermined width in the transport direction;
a process in which
   a plurality of elastic members are placed so that a part of each of the elastic members overlaps the adhesion area,
   the elastic members being stretched in the transport direction;
a process in which
   the lower bandlike member and the upper bandlike member are stacked such that the elastic members are sandwiched between the lower and upper bandlike members, and
   the upper bandlike member adheres to the lower bandlike member and the elastic members on the adhesion area;
a process in which
   the lower bandlike member and the upper bandlike member are folded on a center portion in an intersecting direction so that the upper bandlike member is located inside the lower bandlike member,
   the intersecting direction intersecting the transport direction;
a process in which
   a welded section on which the folded lower bandlike member and the folded upper bandlike member are to be welded to each other is formed on a second area,
   the second area being located on both sides of the first area in the transport direction and having a predetermined width in the transport direction; and
a process in which
   the lower bandlike member, the upper bandlike member and the elastic members are cut together on the cutting position, and
   the lower exterior member and the upper exterior member which are shaped in the form of underpants are cut and separated from the lower bandlike member and the upper bandlike member.

With such a method for manufacturing an absorbent article, it is possible to manufacture a pull-on diaper in which the welding strength of the side parts thereof is sufficient and whose appearance is good.

In such a method for manufacturing an absorbent article, the second area does not overlap the first area within at least a partial area along the transport direction.

With such a method for manufacturing an absorbent article, an area in which the adhesion area and the welded section do not overlap is formed. This can prevent the following phenomena: welding strength of the welded section is insufficient due to the oil of adhesive which forms the adhesion area; and the number of maintenance operations of a welding device increases due to putting of adhesive.

In such a method for manufacturing an absorbent article, it is preferable that the adhesive is not put on the second area.

With such a method for manufacturing an absorbent article, the welded section can be formed on an area on which adhesive is not put. This can achieve sufficient welding strength of the welded section. In addition, since the elastic members do not adhere to the substrate of a diaper in the area, the elastic members are more likely to contract. The contraction of the elastic members allows the substrate to shrink more easily, and this reduces the width of side areas of a pull-on diaper, which extend beyond both transverse end sections. This can improve the appearance of a diaper 1.

In such a method for manufacturing an absorbent article, it is preferable that the adhesive is put on the second area, and that an amount per unit area of the adhesive that has been put on the second area is smaller than an amount per unit area of the adhesive that has been put on the first area.

With such a method for manufacturing an absorbent article, the welded section can be formed on an area on which a small amount of adhesive is put (having a small basis weight). This makes it easier to ensure sufficient welding strength of the welded section. In addition, the elastic members are less likely to adhere to the substrate on the second area. Accordingly, the elastic members and the substrate become more likely to shrink, and this reduces the width of side areas of a pull-on diaper, which extend beyond both transverse end sections. This can improve the appearance of a diaper 1.

In such a method for manufacturing an absorbent article, it is preferable that the adhesive is put on the second area, and that an amount per unit area of the adhesive which is put on the first area and the second area is determined so that welding strength in a case in which the lower bandlike member and the upper bandlike member are welded in the second area on which the adhesive is put is larger than welding strength in a case in which the lower bandlike member and the upper bandlike member are welded in the first area on which the adhesive is put.

With such a method for manufacturing an absorbent article, even if adhesive is put on the second area, it is easy to ensure sufficient strength of the welded section formed on the second area. In addition, the elastic members are less likely to adhere to the substrate on the second area. Accordingly, the elastic members and the substrate become more likely to shrink, and this reduces the width of side areas of a pull-on diaper, which extend beyond both transverse end sections. This can improve the appearance of a diaper 1.

In such a method for manufacturing an absorbent article, it is preferable that an amount per unit area of the adhesive that has been put on the second area is smaller than an amount per unit area of the adhesive that has been put on the first area.

With such a method for manufacturing an absorbent article, the welded section can be formed on an area on which a small amount of adhesive is put (having a small basis weight). This makes it easier to ensure sufficient welding strength of the welded section. In addition, the elastic members are less likely to adhere to the substrate on the second area. Accordingly, the elastic members and the substrate become more likely to shrink, and this reduces the width of side areas of a pull-on diaper, which extend beyond both transverse end sections. This can improve the appearance of a diaper 1.

In such a method for manufacturing an absorbent article, it is preferable that a plurality of the welded sections are formed at intervals along the intersecting direction, and that a distance between two welded sections that are formed adjacent in the intersecting direction is larger than a diameter of the elastic member.

With such a method for manufacturing an absorbent article, the elastic members enter between two adjacent welded sections. Accordingly, the welded sections become less likely to be placed on the elastic members, and this makes it easier to ensure sufficient welding strength of the welded sections. In addition, the elastic members are firmly held being sandwiched between the upper bandlike member and the lower bandlike member. Accordingly, problems such as elastic drop-off can become less likely to occur.

In such a method for manufacturing an absorbent article, it is preferable that the process in which the adhesion area is formed is performed by placing the elastic members on at least either one of the lower bandlike member and the upper bandlike member, adhesive being applied to the elastic members on a certain area while the elastic members being stretched in the transport direction.

With such a method for manufacturing an absorbent article, applying the adhesive to the elastic members increase adhesion of the elastic members on the adhesion area compared to the cases where the adhesive is not applied to the elastic members. This makes it easier to prevent problems such as elastic drop-off. Further, the process in which the adhesion area is formed and the process in which the elastic members are placed are simultaneously performed, and this allows the absorbent articles to be efficiently manufactured.

### === First Embodiment ===

### < Basic Configuration of Diaper 1 >

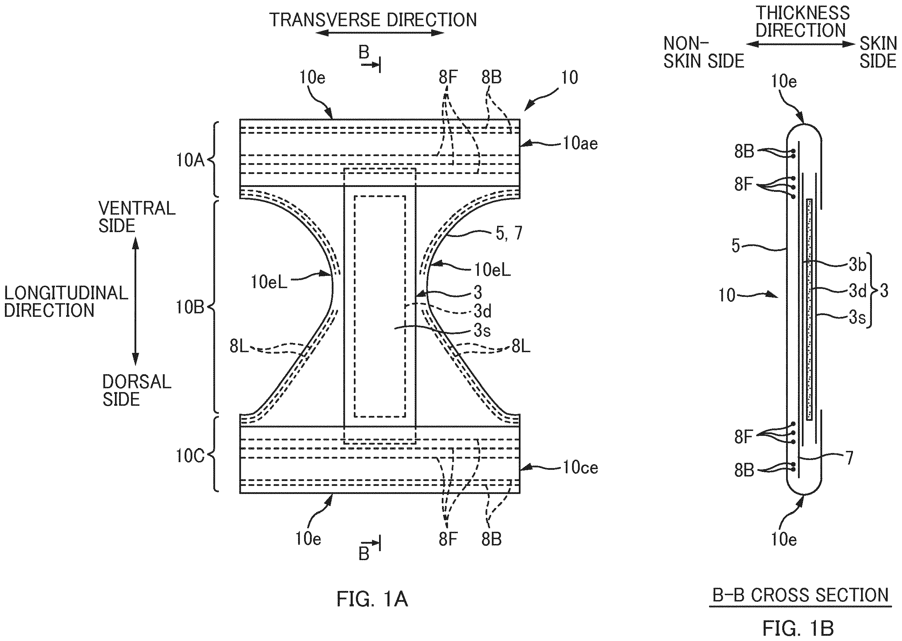

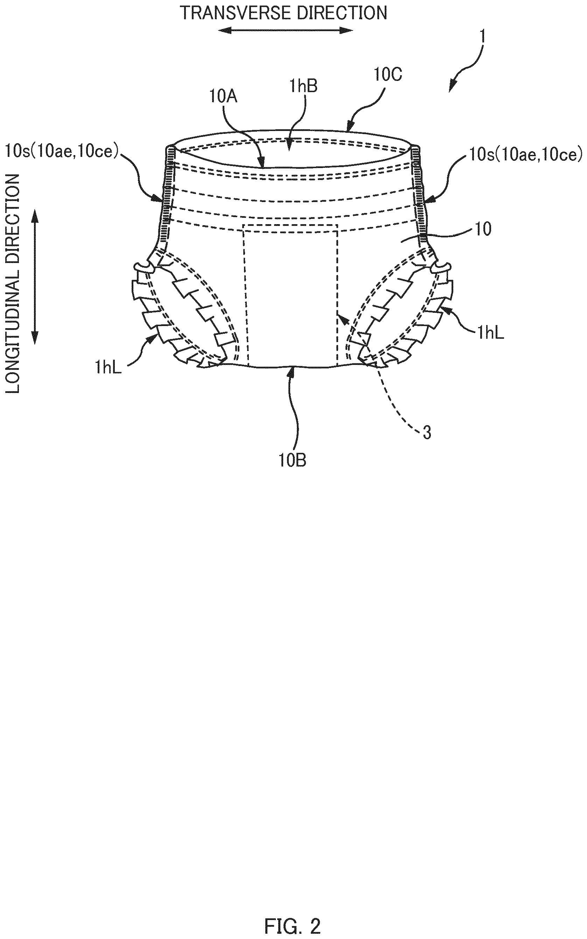

As an example of an absorbent article manufactured in a manufacturing method of the present embodiment, a pull-on disposable diaper (hereinafter referred to as a diaper 1) is employed and the basic configuration thereof will be described first. FIG. 1A is a plan view of a disposable diaper 1 which is spread out and FIG. 1B a cross-sectional view taken along line B-B in FIG. 1A. FIG. 2 is a schematic perspective view of a diaper 1 which is shaped in the form of underpants.

As shown in FIGS. 1A and 1B, the diaper 1 which is spread out has the longitudinal direction, the transverse direction (a direction which intersects the longitudinal direction) and the thickness direction (a direction which intersects the longitudinal direction and the transverse direction). The diaper 1 includes the following components: a lower exterior member 5 (herein also referred to as an outer back) located on the side towards a wearer's garment; an upper exterior member 7 (herein also referred to as an outer top) which is stacked in the thickness direction on and is joined to the lower exterior member 5 from the side towards a wearer's skin; and a liquid-absorbent, absorbent main body 3 which is stacked in the thickness direction on and is joined to the upper exterior member 7 from the side towards the wearer's skin, and which absorbs excrement such as urine.
The lower exterior member 5 and the upper exterior member 7 are flexible stretchable members in the form of sheet, and are made of materials such as nonwoven fabric. As shown in FIG. 1B, the longitudinal length of the lower exterior member 5 is longer than the longitudinal length of the upper exterior member 7, and the diaper 1 which is spread out has a configuration in which the lower exterior member 5 is folded back on longitudinal edge sections 10e to wrap the longitudinal end areas of the absorbent main body 3. In the following description, the lower exterior member 5 and the upper exterior member 7 which is stacked on the lower exterior member 5 are collectively referred to as an exterior member 10.

The exterior member 10 forms the contour of diaper 1 in the spread-out state, and the planar shape thereof is a substantially hourglass shape (see FIG. 1A). That is, the exterior member 10 (the lower exterior member 5 and the upper exterior member 7) has a shape in which the transverse edges are narrowed transversely inwardly in the longitudinal central section. The exterior member 10 is divided along the longitudinal direction as follows: a ventral part 10A that covers a wearer's abdomen; a crotch part 10B that covers a wearer's crotch; and a dorsal part 10C that covers a wearer's back. The diaper 1 which is spread out is two-folded on a substantially center in the longitudinal direction, and the ventral part 10A and the dorsal part 10C are connected on the transverse edges. Thereby, a pull-on diaper 1 having the waist opening 1hB and a pair of leg openings 1hL and 1hL shown in FIG. 2 is formed.

The absorbent main body 3 is in a substantially rectangular shape when viewed from above. The absorbent main body 3 is placed in the center of the transverse direction, and the lengthwise direction of the absorbent main body 3 is along the longitudinal direction of the diaper 1. The absorbent main body 3 does not have to be in a rectangular shape shown in FIG. 1A, and may be, for example, a substantially hourglass shape similar to the exterior member 10. The absorbent main body 3 includes: an absorbent body 3d which absorbs and holds fluid; a liquid-permeable top face sheet 3s which wraps the absorbent body 3d from a wearer's skin side and through which excrement such as urine passes; and a liquid-impermeable back face sheet 3b made of materials such as film which wraps the absorbent body 3d from the non-skin side and which prevents fluid leakage from the non-skin side. The absorbent body 3d is made by shaping fluid-absorbent fiber such as pulp fiber into a certain shape such as a substantially rectangular parallelepiped. And, the absorbent body 3d contains superabsorbent polymer therein. The absorbent body 3d may be covered with tissue paper. Further, upstanding gather (leak-proof cuff) for preventing side leakage may be provided on both transverse edges of the absorbent main body 3.

On appropriate positions in the exterior member 10, a plurality of the elastic members 8B, 8F, 8L ..., which are made of rubber thread and the like, are placed between the lower exterior member 5 and the upper exterior member 7. These elastic members 8B, 8F, 8L ... are for providing stretchability to a diaper 1. For example, in the longitudinal edge sections 10e and 10e of the exterior member 10, a plurality of the waist elastic members 8B and 8B are respectively disposed along the transverse direction; the longitudinal edge sections 10e and 10e are respectively an edge section 10e which forms the waist opening 1hB in the ventral part 10A (corresponding to a waist-circumference edge section) and an edge section 10e which forms the waist opening 1hB in the dorsal part 10C (corresponding to a waist-circumference edge section). These waist elastic members 8B and 8B are stretched in the transverse direction and partial areas thereof adhere and are fixed to the lower exterior member 5 and the upper exterior member 7. And stretchability is thereby provided to the waist opening 1hB, which is composed of the edge sections 10e and 10e. Similarly, a plurality of fitting-gather elastic members 8F, 8F, ... are disposed along the transverse direction at the position near the center in the longitudinal direction with respect to the positions of the waist elastic members 8B. These fitting-gather elastic members 8F, 8F, ... are stretched in the transverse direction. And, partial areas of thereof adhere and are fixed to the lower exterior member 5 and the upper exterior member 7. Stretchability in the transverse direction is thereby provided to parts between the waist opening 1hB and the longitudinal center. In addition, a plurality of the leg elastic members 8L, 8L, ... are respectively disposed of edge sections 10eL and 10eL of the transverse edge sections of the exterior member 10, the edge sections 10eL and 10eL being to form the leg openings 1hL. These leg elastic members 8L, 8L, ... are stretched, and partial areas of thereof adhere and are fixed to the lower exterior member 5 and the upper exterior member 7. Stretchability is thereby provided to the edge sections 10eL and 10eL, which are to form the leg openings 1hL and 1hL.

A pull-on diaper shown in FIG. 2 is shaped by folding back the crotch part 10B of the exterior member 10 near the longitudinal center and by joining (sealing) the transverse end section 10ae of the ventral part 10A and the transverse end section 10ce of the dorsal part 10C. These joined transverse end section 10ae and transverse end section 10ce are to be the end sections 10s of the diaper 1. In the present embodiment, the end sections 10s contract inwardly in the transverse direction (towards the center in the transverse direction) and beyond-end parts of the end sections 10s in the transverse direction become less likely to be noticeable on the side areas of the diaper 1. This makes it possible to prevent the deterioration of the appearance of the diaper 1.

### < Method for Manufacturing Diaper 1>

A method for manufacturing diapers 1 associated with the present embodiment will be described.

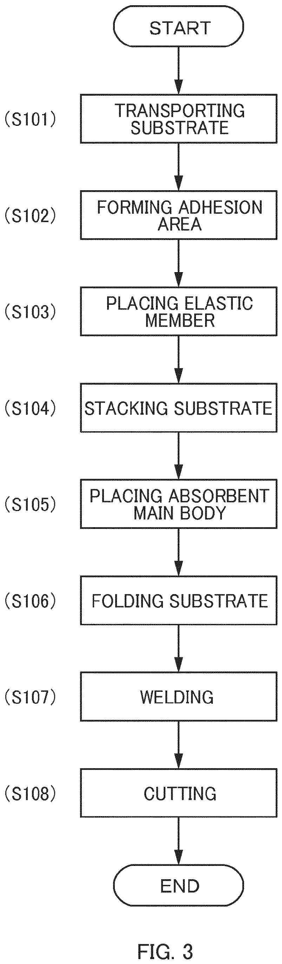

FIG. 3 is a flow chart of processes for manufacturing diapers 1 of the first embodiment. The diapers 1 are continuously manufactured by performing the processes represented by the symbols S101 to S108 of the manufacturing line of the diapers 1 in FIG. 3. The processes will be sequentially described below.

In the manufacturing line of the diaper 1, the substrate of the diapers 1 is transported by a suitable transport mechanism along a certain transport direction at a certain transport speed (S101).

The "substrate of the diapers 1" herein means a band-like member in which a plurality of the lower exterior members 5 (or the upper exterior members 7) shown in FIG. 1A are continuously linked in the transverse direction. Hereinafter, the band-like member in which the lower exterior members 5 continue in the transverse direction is referred to as a lower bandlike member 15, and the band-like member in which the upper exterior member 7 continue in the transverse direction is referred to as an upper bandlike member 17. The "transport direction" is a direction along the transverse direction of the exterior member 10 in FIG. 1A. In the following description, a direction which intersects the transport direction (that is, a direction along the longitudinal direction of the exterior member 10) is referred to as the "intersecting direction". In the first embodiment, the processes S102 to S108 are performed while the lower bandlike member 15 is transported in the transport direction.

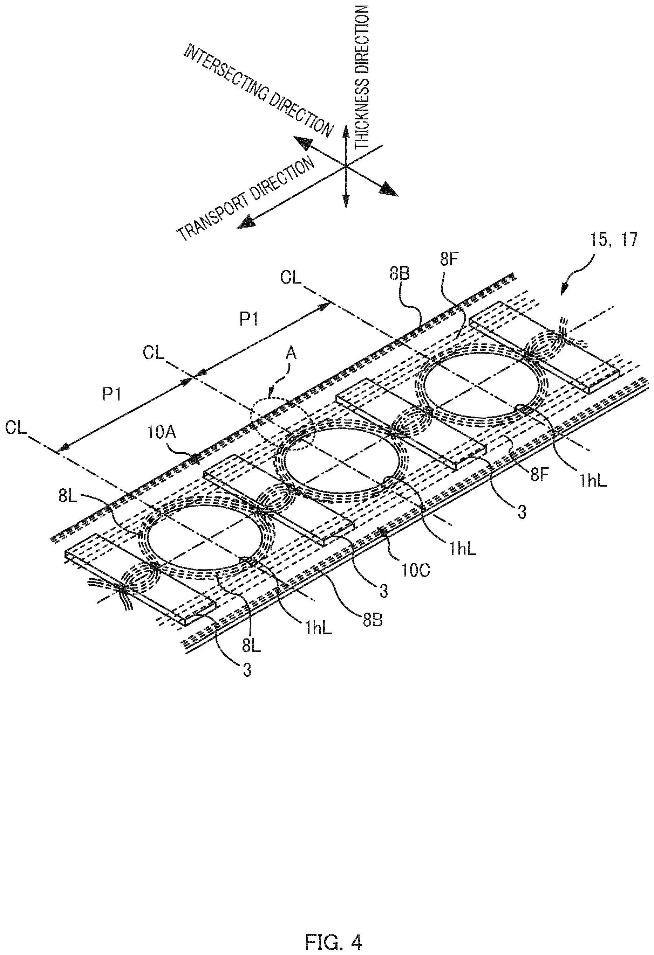

FIG. 4 is a diagram illustrating how the substrate of the diapers 1 is transported in the transport direction. FIG. 4 shows how the lower bandlike member 15, the upper bandlike member 17, the elastic members 8B, 8F, and 8L and the absorbent main body 3 are transported in the processes of S101 to S105; the lower bandlike member 15 and the upper bandlike member 17 are stacked such that the elastic members 8B, 8F, and 8L are sandwiched between them, and the absorbent main body 3 is joined thereon. The following section describes how the elastic members 8B, 8F, and 8L and the upper bandlike member 17 are stacked onto the lower bandlike member 15 which is being transported. But, it is also acceptable that the upper bandlike member 17 is transported and the lower bandlike member 15, etc. are stacked onto the upper bandlike member 17 which is being transported.

The lower bandlike member 15 is transported in the transport direction in a state where the lower exterior members 5 which are adjacent to each other in the transport direction (the transverse direction) continue on a cutting position CL. The cutting position CL is a reference position when the lower exterior member 5 (the exterior member 10) is cut and separates from the lower bandlike member 15 in the following cutting process (S108). That is, the cutting position CL is a position on which a cutting line is formed, the cutting line being for cutting diapers 1 from the bandlike member into a single piece, and also the cutting position CL is for serving as the end sections 10s of a diaper 1 which is shaped in the form of underpants (see FIG. 2). Also, the cutting line is a reference position in the following adhesion-area forming process (S102) and welding process (S107). Accordingly, in the present embodiment, the operations of the processes are controlled based on the cutting position CL. In the present embodiment, the cutting positions CL are determined at intervals (pitches) P1 in the transport direction.

While the lower bandlike member 15 being transported, the leg openings 1hL are formed. The leg openings 1hL are formed in an area extending in the transport direction and being centered on the cutting position CL, and are each indicated as the edge section 10eL of the exterior member 10 in FIG. 1A. In the present embodiment, the leg openings 1hL are formed using a cutting device (not shown) at the same time as the position of the cutting position CL has been determined or at a certain timing thereafter.

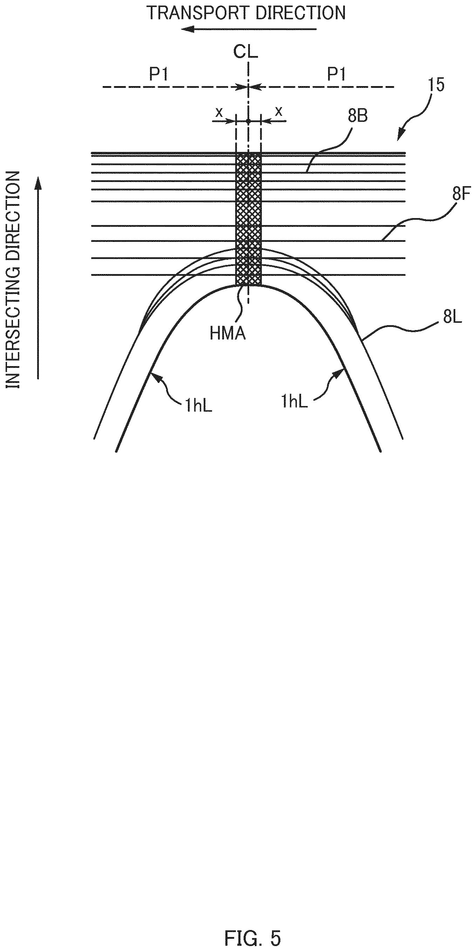

Next, adhesion areas are formed by putting adhesive onto certain areas on the lower bandlike member 15 which is being transported in the transport direction (S102). FIG. 5 is illustrating a first area and an adhesion area. FIG. 5 is magnified views of the area A in FIG. 4, and shows the substrate of a diaper 1 while its cutting position CL being centered. Though the elastic members 8B, 8F, and 8L are placed on the lower bandlike member 15 in FIG. 5, the elastic members will in fact be placed in the next process (S103). Accordingly, when adhesive is put on the lower bandlike member 15 in the adhesion-area forming process S102, the elastic members 8B, 8F, and 8L have not placed on the lower bandlike member 15 yet.

In adhesion-area forming process (S102), the adhesion areas HMA are formed on the first area by putting adhesive thereon, and the first area is a certain area on the substrate. Specifically speaking, the adhesion areas HMA are formed by applying adhesive to the first area on the surface of the lower bandlike member 15 using an adhesive coater (not shown). Hot-melt type adhesive is used as an example of the adhesive for forming the adhesion areas. As indicated by the hatched area in FIG. 5, the first area includes a cutting position CL within the hatched area, and has a predetermined width in the transport direction. In the example of FIG. 5, the first area is a rectangular area elongated along the intersecting direction, its center is located at the cutting position CL, and having the width of 2x in the transport direction. The adhesion areas HMA are formed by applying adhesive to the entire area of the first area. That is, in the case of FIG. 5, the adhesion area HMA and the first area are identical. But, it is not necessary to apply adhesive throughout the entire area of the first area. The adhesion area HMA may be formed in a certain range within the first area. The width 2x of the first area in the transport direction and the size of the adhesion area HMA (the width in the transport direction) are determined so that elastic members 8B, etc. (to be described later) which extend in the transport direction can be firmly attached and held, and so that the transverse end sections 10s are not excessively large at final stage of a diaper 1 shown in FIG. 2.

Adhesive may be applied onto areas other than the first area. But, it is desirable that adhesive is not applied onto a second area (to be described later), or that a smaller amount per unit area of adhesive is applied onto the second area than that of the first area (to reduce basis weight per unit area). This reason will be described later. In the foregoing description, the adhesion areas HMA are formed on the lower bandlike member 15 by putting adhesive on the lower bandlike member 15. However, the adhesion areas HMA may be formed on the upper bandlike member 17 by putting adhesive on the upper bandlike member 17. Also, the adhesion areas HMA may be formed on both members by putting adhesive on both of the lower bandlike member 15 and the upper bandlike member 17.

After the adhesion areas HMA are formed, the elastic members 8B, 8F, and 8L are placed (S103). As shown in FIG. 4, while being stretched in the transport direction, a plurality of the waist elastic members 8B are placed in an area of each end section of the lower bandlike member 15 (or the upper bandlike member 17) in the intersecting direction. Then, each of the elastic members 8B is placed so that at least a partial area thereof overlap the adhesion area HMA (corresponding to the hatched part in FIG. 5) which has been formed in S102. Similarly, while being stretched in the transport direction, a plurality of the fitting-gather elastic members 8F are placed in an inner area of the lower bandlike member 15 (the upper bandlike member 17) with respect to each end section in the intersecting direction. Then, each of the fitting-gather elastic members 8F is placed so that at least a partial area thereof overlap the adhesion area HMA which has been formed in S102. Further, a plurality of the leg elastic members 8L are placed in a substantially arcuate manner along the leg opening 1hL of the lower bandlike member 15. While the leg elastic members 8L being stretched along the leg opening 1hL (that is, being stretched along the leg opening in a direction having a component parallel to the transport direction), the leg elastic members 8L are placed so that a partial area thereof overlap the adhesion area HMA which has been formed in S102. Each of the elastic members 8B, 8F and 8L thereby adheres to the lower bandlike member 15 (the upper bandlike member 17) on an area in which the elastic member is stacked on the adhesion area HMA. However, in the process of S102, the elastic members are each merely placed on the adhesion area HMA, and at this stage, the elastic members 8B, 8F, and 8L are not fixed to the lower bandlike member 15 completely.

After the elastic members are placed, the upper bandlike member 17 are stacked on the lower bandlike member 15 and the elastic members 8B, 8F, and 8L which are placed on the lower bandlike member 15 (S104). Then, by pressurizing in the thickness direction so that the upper bandlike member 17 is pressed against the lower bandlike member 15, the upper bandlike member 17 adheres to the lower bandlike member 15 on the adhesion areas HMA. The elastic members 8B, 8F, and 8L are thereby held while being sandwiched in the thickness direction between the lower bandlike member 15 and the upper bandlike member 17, and the exterior members of the diapers 1 are formed. The length of the lower bandlike member 15 in the intersecting direction is longer than the length of the upper bandlike member 17 in the intersecting direction, but this is not shown in FIG. 4. Accordingly, at the stage of S104, both end sections of the lower bandlike member 15 in the intersecting direction extend beyond both end sections of the upper bandlike member 17 in the intersecting direction.

As mentioned above, the elastic members 8B, 8F, and 8L are placed being stretched. And, in this process, these elastic members 8B, 8F, and 8L are sandwiched between and firmly held by the lower bandlike member 15 and the upper bandlike member 17. This allows each of the elastic members 8B, 8F, and 8L to produce stretchability in the lower bandlike member 15 and the upper bandlike member 17. For example, the fitting-gather elastic members 8F can produce stretchability between two adhesion areas HMA which are adjacent in the transport direction. In the present embodiment, since the adhesion areas HMA are formed about the cutting position CL, the fitting-gather elastic members 8F produce stretchability in the transport direction in an area of the pitch P1 between a cutting position CL and an adjacent cutting position CL (more precisely, in an area between the cutting position CL and a position distant x in the transport direction in FIG. 5).

Subsequently, the absorbent main body 3 is placed on and joined to the upper side of the upper bandlike member 17 in the thickness direction, the upper bandlike member 17 being stacked on and adhering to the lower bandlike member 15 (S105). Note that the absorbent main body 3 is separately manufactured in a manufacturing line for absorbent main bodies (not shown). As shown in FIG. 4, the absorbent main body 3 is placed at a position between the cutting positions CL in the transport direction (that is, the transverse central section of the exterior member 10) so that the lengthwise direction of the absorbent main body 3 is in the intersecting direction. And, the absorbent main body 3 is joined to the surface of the upper bandlike member 17 with adhesive and the like. After the absorbent main body 3 is joined, a beyond-edge part of end section of the lower bandlike member 15 in the intersecting direction is bent so as to wrap the absorbent main body 3 from the upper side in the thickness direction (see FIG. 1B). Parts of the lower bandlike member 15 adhere to the absorbent main body 3 and/or the upper bandlike member 17.

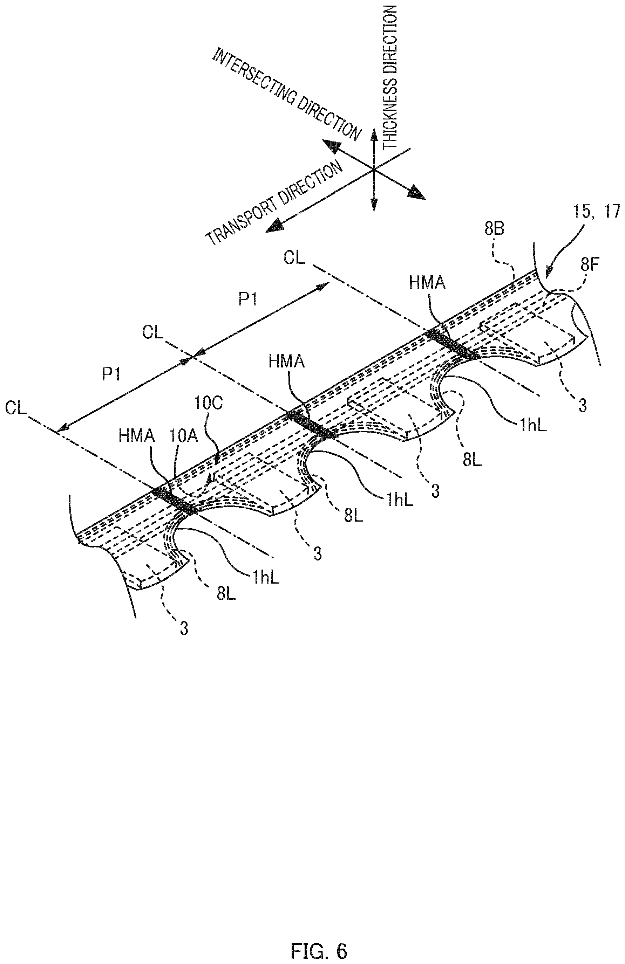

Subsequently, the lower bandlike member 15 and the upper bandlike member 17 which are stacked are folded on the center in the intersecting direction so that the upper bandlike member 17 is located inside the lower bandlike member 15 (that is, inside the upper bandlike member 17 in the thickness direction) (S106). FIG. 6 is a diagram illustrating how the folded substrate of diapers 1 is transported after the process of S105. As shown in the figure, by folding on the center in the intersecting direction, the ventral part 10A and the dorsal part 10C of the diaper 1 are stacked in the thickness direction and are in a state in which pants-form substrates continue in the transport direction. In this process, the bandlike members and the absorbent main body 3 are merely bent and stacked, and the ventral part 10A and the dorsal part 10C are not joined to each other.

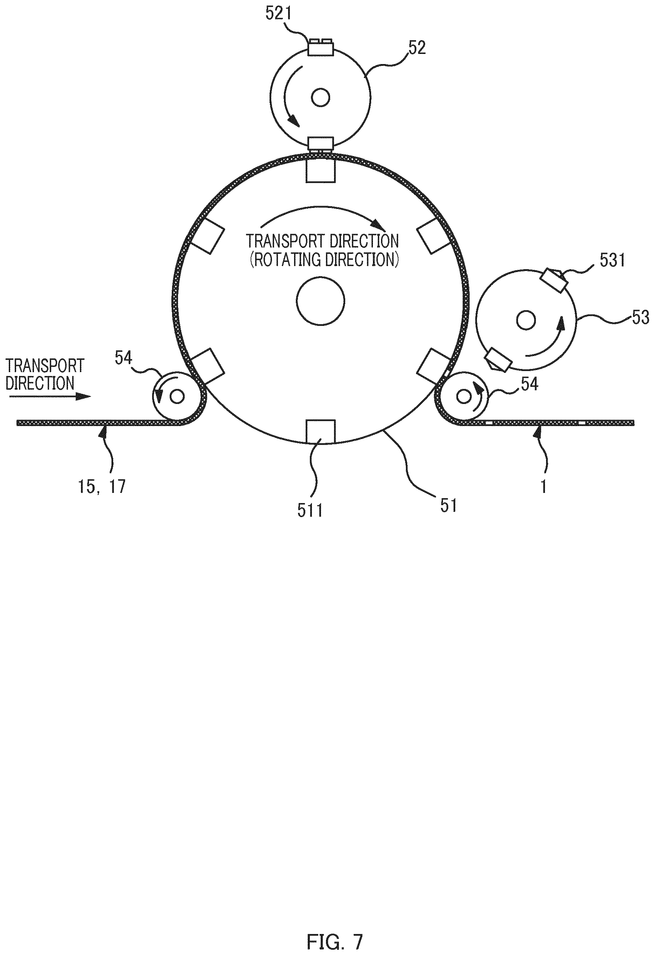

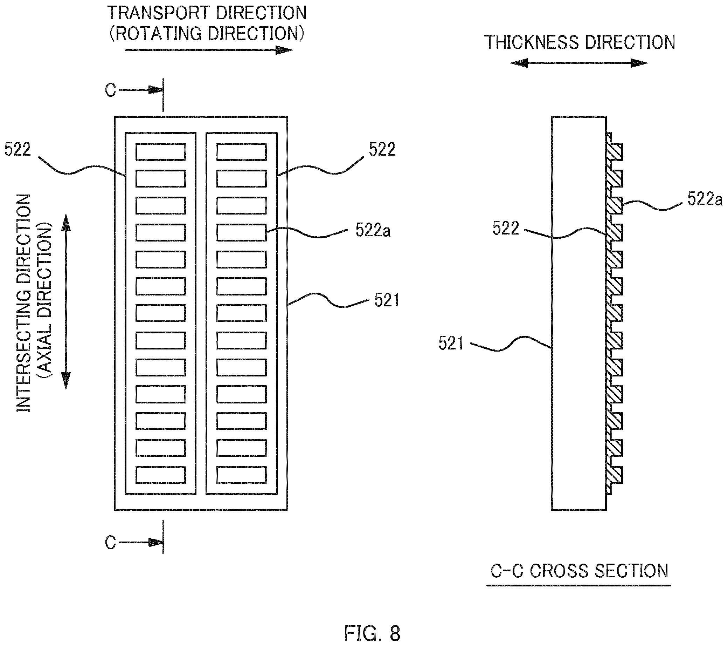

Subsequently, the ventral part 10A and the dorsal part 10C which are stacked are welded onto a certain area (S107). A method of the welding is heat welding or ultrasonic welding. In the following description, an example of heat welding will be described. FIG. 7 is a schematic side view of a welding-and-cutting apparatus 50 used in the manufacturing line of the first embodiment. FIG. 8 is a diagram illustrating a heating block 521 of the welding-and-cutting apparatus 50. With the welding-and-cutting apparatus 50, cutting in the next process (S108) is performed as well as welding of the ventral part 10A and the dorsal part 10C.

The welding-and-cutting apparatus 50 includes: an anvil roll 51; a sealing roll 52; a cutter roll 53; and transportation-assisting rolls 54. The anvil roll 51 is a drum-like rotating body, and has an sucking mechanism (not shown) on the circumferential surface thereof. The anvil roll 51 rotates while the upper bandlike member 17 and the lower bandlike member 15 are sucked on and fixed to the circumferential surface thereof, and thereby the bandlike members 15 and 17 are transported in the rotating direction. On the circumferential surface of the anvil roll 51, a plurality of anvil brocks 511 are provided. Each of the anvil brocks 511 is a member elongated in the axial direction of the anvil roll 51 (corresponding to the foregoing intersecting direction). A substrate can be welded by being sandwiched and heated between the anvil brock 511 and the following heating block 521 in the direction of the normal of the anvil roll 51 (corresponding to the foregoing thickness direction). In addition, the substrate can be cut by being sandwiched and pressed between the anvil brock 511 and the following cutter block 531 in the direction of the normal of the anvil roll 51. In the figure, the anvil brocks 511 are provided in the positions which divide along the circumferential direction the circumference of the anvil roll 51 into six equal parts, but the number of the anvil brocks 511 included in a single anvil roll 51 is not limited to six.

The sealing roll 52 is a rotating body placed at a position facing the anvil roll 51 as shown in FIG. 7. On the circumferential surface of the sealing roll 52 (corresponding to the intersecting direction), the heating block 521 is provided along the axial direction of the sealing roll 52. These components have a positional relationship in which the anvil brock 511 and the heating block 521 face periodically by rotation of the anvil roll 51 and simultaneous rotation of the sealing roll 52 in the opposite direction. In the present embodiment, adjustment is made so that the anvil brock 511 and the heating block 521 face each other at a point-in-time when the bandlike members 15 and 17 are transported by pitch P1 between adjacent two cutting positions CL in the transport direction. In FIG. 8, though the heating blocks 521 are provided respectively in the positions which divide along the circumferential direction the circumference of the sealing roll 52 into two equal parts, the number of the heating blocks 521 is not limited to two. Each of the heating blocks 521 includes two protruding sections 522 aligned in the rotating direction (the transport direction). On each protruding section 522, a plurality of rectangular portions 522a protruding in the thickness direction are placed at intervals along the axial direction (the intersecting direction). When the rectangular portions 522a face the anvil brock 511 of the anvil roll 51 and come into contact with the substrates, the substrates are heated by the heated rectangular portion 522a and the anvil brock 511. And this makes it possible to heat-weld (heat-seal) the areas corresponding to the rectangular portions 522a. The shape of the rectangular portions 522a is not limited to the example shown in FIG. 7, and does not have to be rectangle. For example, the shape of the rectangular portions 522a may be triangle or circle. In addition, the number of the rectangular portions 522 may change depending on the area of the welding range or welding conditions as appropriate. However, in FIG. 8, it is preferable that a gap between two adjacent rectangular portions 522a in the axial direction (the intersecting direction) (that is, a gap in the intersecting direction) is larger than the diameter of the elastic members 8B and 8F. This reason will be described later.

In the foregoing example, heat-welding (heat-sealing) is described. Though the configuration of apparatuses for ultrasonic welding (sonic-sealing) is partly different therefrom (e.g., the heating block 521 is replaced with ultrasonic horn), the basic operations such as transportation operation of the welding-and-cutting apparatus 50 are the same.

The cutter roll 53 is used, in the next cutting process (S108), when cutting the lower bandlike member 15 and the upper bandlike member 17 on a part of the cutting position CL along the intersecting direction. The cutter roll 53 is a rotating body placed at a position facing the anvil roll 51 and downstream from the sealing roll 52 in the transport direction. On the circumferential surface of the cutter roll 53, the cutter block 531 is provided along the axial direction of the cutter roll 53 (corresponding to the intersecting direction). These components have a positional relationship in which the anvil brock 511 and the cutter block 531 faces periodically by rotation of the anvil roll 51 and simultaneous rotation of the cutter roll 53 in the opposite direction. The cutter block 531 includes a cutter (not shown) along the axial direction (the intersecting direction). When the cutter faces the anvil brock 511 of the anvil roll 51 and comes into contact with a substrate, the substrate can be cut in the intersecting direction.

The transportation-assisting rolls 54 are provided upstream and downstream from the anvil roll 51 in the transport direction. The transportation-assisting rolls 54 assist the transportations of the lower bandlike member 15 and the upper bandlike member 17 along the circumferential surface of the anvil roll 51, and adjusts the magnitude of tension which is exerted on the lower bandlike member 15 and the upper bandlike member 17 which are being transported. Uniform tension during transportation makes it possible to prevent the occurrence of faulty weld and/or displacement at the time of welding.

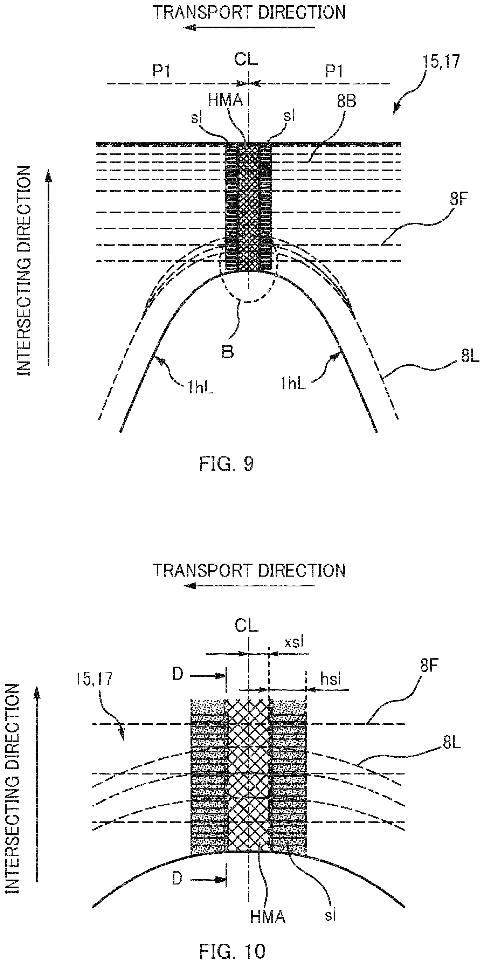

Here, the description return again to the welding process (S107). FIG. 9 is a diagram illustrating areas where welded section is formed in welding process, and shows the areas corresponding to those of FIG. 5. FIG. 10 is a magnified view of the area B in FIG. 9, and illustrates second areas and the welded sections. While the lower bandlike member 15 and the upper bandlike member 17 being transported along the circumferential surface of the anvil roll 51 of the welding-and-cutting apparatus 50, the members 15 and 17 are welded on the certain area at a point-in-time when the anvil brock 511 is positioned facing the heating block 521. And, the welded sections sl shown in FIGS. 9 and 10 are formed.

The welded sections sl are formed within the second areas according to the shape of the protruding section 522 of the foregoing heating block 521. The second areas are areas located on both sides of the first area in the transport direction, and each has a predetermined width in the transport direction. In FIG. 10, the second areas are indicated as colored areas. A pair of the second areas are placed on both sides of the cutting position CL, and are defined in substantially rectangular areas whose width in the transport direction is a value hsl at positions distant xsl in the transport direction from the cutting position CL. In the present embodiment, within the second areas, a plurality of rectangular welded sections sl are formed at intervals along the intersecting direction.

Each of the second areas are defined so that a part thereof overlaps the first area in the transport direction. Specifically speaking, the second area is defined so that the distance xsl from the cutting position CL to an end section of the second area (the end section closer to the cutting position CL) in the transport direction is smaller than the distance x (see FIG. 5) from the cutting position CL to an end section of the first area in the transport direction (x > xsl). In other words, the end section of the first area in the transport direction overlaps the end section of the second area in the transport direction. Accordingly, in some cases, a part of the welded section sl formed in the second area overlaps, in the transport direction, the adhesion area HMA formed in the first area, as shown in FIG. 10.

The welded sections sl and the adhesion areas HMA are formed overlapping on partial areas, and this enables the elastic members 8L, 8B and 8F to be fixed more firmly, the elastic members 8L, 8B and 8F being stretched and adhering to the lower bandlike member 15 (the upper bandlike member 17) in the adhesion areas HMA. For example, when the fitting-gather elastic members 8F (hereinafter merely referred to as "elastic members 8F") are cut at a cutting position CL in the following cutting process (S108), those elastic members 8F contract in the transport direction from a portion which has been cut. At this stage, if the elastic members 8F insufficiently adhere to the lower bandlike member 15 (the upper bandlike member 17), the elastic members 8F are removed (elastic drop-off) from the adhesion areas HMA; consequently, the elastic members 8F cannot provide stretchability to the lower bandlike member 15 (the upper bandlike member 17). This causes a problem such that fitting around the wearer's waist deteriorate when a diaper 1 is worn. As opposed thereto, the welded sections sl are formed overlapping some parts of the adhesion areas HMA as in the present embodiment. Thereby, the elastic members 8F adhere to the lower bandlike member 15 (the upper bandlike member 17) on the adhesion areas HMA. In addition thereto, the elastic members 8F become more likely to be held while being sandwiched between the lower bandlike member 15 and the upper bandlike member 17 which are pressurized by the welded sections sl in the thickness direction. This makes the elastic members 8F to be less likely to be removed.

It is desirable that an overlapping part of the welded sections sl and the adhesion areas HMA in the transport direction is as small as possible. The reason is that, if the welded sections sl are formed so as to overlap the entire areas of the adhesion areas HMA, welding strength of the welded sections sl may be insufficient due to the oil of hot-melt adhesive which forms the adhesion areas HMA. Putting of hot-melt adhesive onto the heating block 521 used in heat welding and/or the ultrasonic horn used in ultrasonic welding may cause problems such as increase of the number of maintenance operations of the welding-and-cutting apparatus 50, or may prevent continuous manufacturing of the diapers 1. Accordingly, in the present embodiment, the welded sections sl overlap only partial areas of some of the adhesion areas HMA, and this enables the welded sections sl to obtain sufficient welding strength. In the case shown in FIG. 10, the second areas are defined so that distance (xsl + hsl) from the cutting position CL to an end section of the second area (the end section away from the cutting position CL) in the transport direction is larger than the distance x from the cutting position CL to an end section of the first area in the transport direction (x < (xsl + hsl)). Accordingly, the welded sections sl do not overlap the adhesion area HMA in the transport direction within at least a part thereof, and therefore a sufficient welding strength becomes likely to be achieved in the welded sections sl. The "welding strength" herein indicates the tensile strength of the welded section, and can be evaluated by testing a welded band-like member with a tensile strength tester (e.g. a tester described in JIS).

In the present embodiment, there is a case in which adhesive is applied on an area other than the first area. That is, in some cases, adhesive is applied on the second areas. In this case, the amount per unit area (basis weight) of adhesive which is put on the second area is smaller than at least the amount per unit area (basis weight) of adhesive which is put on the first area. This allows the foregoing problems to become less likely to happen, and therefore the welding strength of the welded sections sl formed in the second areas becomes less likely to deteriorate. However, if the welding strength of the welded sections sl formed in the second areas is sufficient, the amount per unit area of adhesive which is put on the second areas may be larger than the amount per unit area of adhesive which is put on the first area. In other words, the amount per unit area of adhesive may be determined so that welding strength when welding is performed in the second areas is larger than welding strength when welding is performed in the first area.

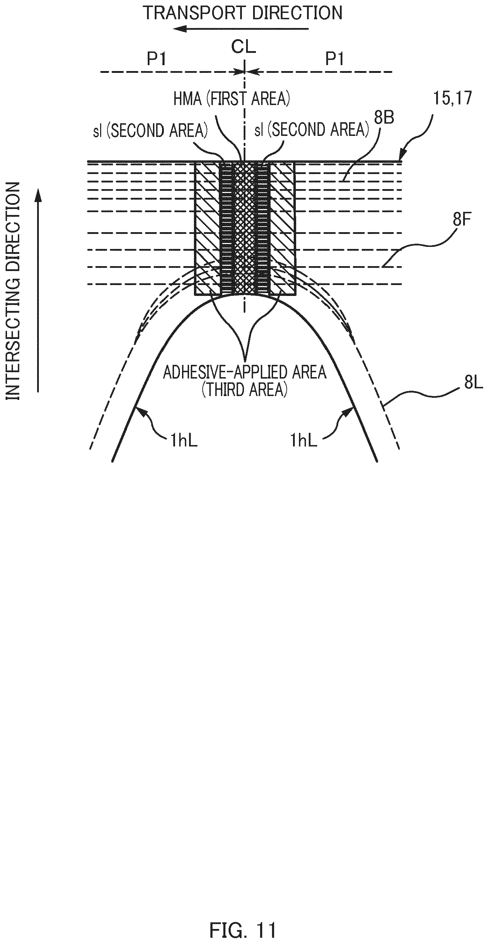

It goes without saying that adhesive may be put on an area other than the first area and the second areas. FIG. 11 is a diagram showing an example of a case in which adhesive is applied to an area except for the first area and the second area. In a case of FIG. 11, as shown in the hatched parts, adhesive is applied to areas outside of the second areas on both sides of the cutting position CL in the transport direction. These areas are defined as third areas, for convenience. The basis weight of adhesive applied to the third areas may be equal to the basis weight of adhesive applied to the first area, or may be larger or smaller than the basis weight to the first area. Since no welded section sl is formed in the third areas, the welded sections sl formed in the second areas have sufficient welding strength regardless of the amount of adhesive applied to the third areas. The sizes and positions of the third areas are not limited to the example of FIG. 11. For example, the third areas may have a larger width in the transport direction than in the case of FIG. 11, and the third areas may be provided at intervals in the transport direction or in the intersecting direction.

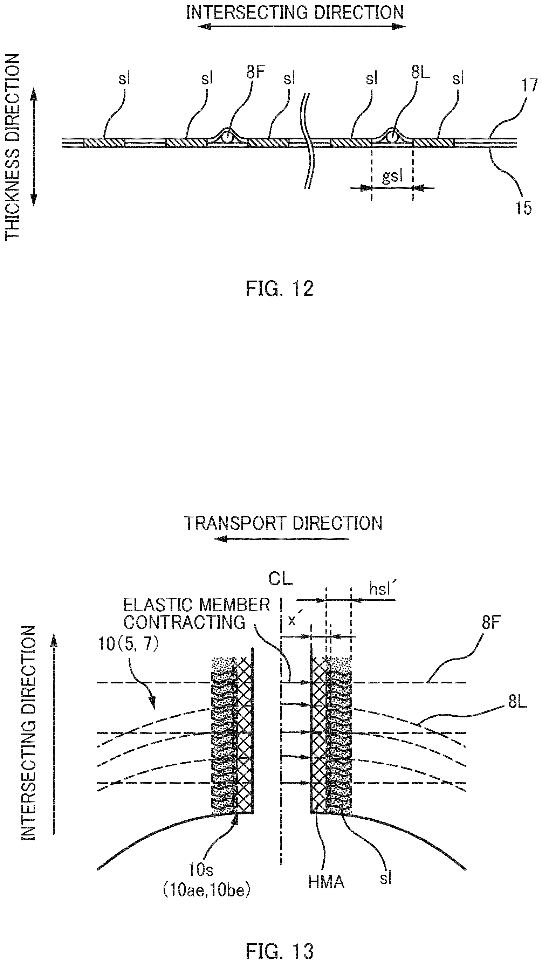

In the present embodiment, the welded sections sl are formed so that the distance (gsl) between two welded sections sl and sl adjacent in the intersecting direction is larger than the diameter (thickness) of each of the elastic members 8B, 8F and 8L. FIG. 12 is a schematic diagram of D-D section in FIG. 10. In FIG. 12, the elastic members 8B, 8F (and 8L) are placed between the welded sections sl and sl adjacent in the intersecting direction while being sandwiched in the thickness direction between the lower bandlike member 15 and the upper bandlike member 17. If the distance gsl between the welded sections sl and sl is smaller than the diameter of the elastic members 8B, 8F and 8L, a welded section sl is formed by welding at a position in the intersecting direction where at least a part of the welded section sl overlaps the elastic members 8B, 8F and 8L. That is, parts of the elastic members 8B, 8F and 8L are welded to the lower bandlike member 15 (the upper bandlike member 17). In this case, the elastic members 8B, 8F and 8L are welded and fixed to the lower bandlike member 15 and the upper bandlike member 17. On the other hand, the elastic members 8B, 8F and 8L exist being sandwiched in the welded sections sl, and this makes insufficient welding strength between the ventral part 10A and the dorsal part 10C. As opposed thereto, as shown in FIG. 12, suppose that the distance gsl between the welded sections sl and sl is larger than the diameter of the elastic members 8B, 8F and 8L. In this case, even when the position where the welded sections sl are to be formed overlaps in the intersecting direction the position where the elastic members 8B, 8F and 8L are placed, if the elastic members 8B, 8F and 8L are pressurized by the anvil brock 511 and the heating block 521 at the time of welding, the elastic members 8B, 8F and 8L move in the intersecting direction in a pushed manner and are displaced to between the welded sections sl and sl. Thus, the position where the welded sections sl are actually formed and the position of the elastic members 8B, 8F and 8L are less likely to overlap in the intersecting direction. This ensures sufficient welding strength in the welded sections sl, and also the elastic members are firmly held being sandwiched between the lower bandlike member 15 and the upper bandlike member 17 which are pressurized in the thickness direction. Accordingly, problems such as elastic drop-off can become less likely to occur.

In the present embodiment, in most area of the second area of the lower bandlike member 15 (the upper bandlike member 17) where the welded sections sl are formed, the surface of nonwoven fabric keeps in good condition. For example, if the adhesion areas HMA are formed within a wide range of the second area or if the surface of nonwoven fabric in the second area is damaged by other means such as a cutter, it is possible that welding is failed which results in insufficient strength of the welded sections sl. But, in the present embodiment, any treatment to damage the surface of nonwoven fabric is not made at least within the second area. Accordingly, sufficient welding strength can be ensured by normal welding.

The lower bandlike member 15 and the upper bandlike member 17 which have been welded are directly transported on the circumferential surface of the anvil roll 51 of the welding-and-cutting apparatus 50 (see FIG. 7), and are cut along the axial direction (the intersecting direction) at a position where the cutter block 531 of the cutter roll 53 faces the anvil brock 511 (S108). In the present embodiment, the lower bandlike member 15, the upper bandlike member 17 and the elastic members 8B, 8F and 8L are cut together along the cutting position CL.

FIG. 13 is a diagram illustrating how the bandlike member and the elastic member are cut in the cutting process (S108), and shows the areas corresponding to those of FIG. 5. In FIG. 13, when cutting along the cutting position CL, the lower exterior member 5 and the upper exterior member 7 which is shaped in the form of underpants are cut and separated from the lower bandlike member 15 and the upper bandlike member 17. Simultaneously, the elastic members 8B, 8F and 8L, which are placed being stretched in the transport direction, contract along the transport direction from the cut parts towards a direction away from the cutting position CL, as indicated by arrows in FIG. 13. In other words, the elastic members 8B, 8F and 8L contract towards the transverse center of a diaper 1. As the elastic members 8B, 8F and 8L contract, the transverse end sections 10s of the lower exterior member 5 and the upper exterior member 7 (see FIGS. 1 and 2) deform so as to shrink towards the transverse center of a diaper 1.

In the present embodiment, while the elastic members 8B, 8F and 8L being stretched in the transport direction and being sandwiched between the lower bandlike member 15 and the upper bandlike member 17, the elastic members are held by the adhesion area HMA formed in the first area. Accordingly, stretching-and-contraction force due to the elastic members 8B, 8F and 8L is exerted on the adhesion area HMA along the transport direction. In this case, if the elastic members 8B, 8F and 8L are cut at the cutting position CL, the adhesion area HMA is pulled by the elastic members which are contracting, and the adhesion area HMA deforms so that the adhesion area HMA itself contracts along the transport direction while moving along the transport direction towards a direction away from the cutting position CL. On the other hand, in the second areas placed on the sides of the first area in the transport direction, adhesive is not put or adhesive is put of a low basis weight with which stretching-and-contraction force is not exerted. And, in the second areas, stretching-and-contraction force is not exerted on the lower bandlike member 15 and the upper bandlike member 17. Accordingly, in the second areas, the elastic members are likely to contract along the transport direction (the transverse direction of a diaper 1), and the lower exterior member 5 and the upper exterior member 7 deform so as to shrink towards the transverse center, by an amount corresponding to the contraction of the elastic members.

This narrows the welded sections sl and the adhesion area HMA which extend beyond both transverse end sections of the exterior member 10 of a pants-shaped diaper 1. Consequently, the welded sections sl and the adhesion area HMA become less likely to looks protruding, and the appearance of the diaper 1 becomes good. For example, in FIG. 13, the first area indicated by the hatched areas deforms so that its width x in the transport direction changes to the width x' (x > x'), and the second areas indicated by the colored areas deform so that the width hsl in the transport direction changes to the width hsl' (hsl > hsl') . If the lower exterior member 5 and the upper exterior member 7 are stretchable members, the exterior member itself contracts and deforms so that the first area and the second areas become narrower in the transport direction. On the other hand, if the lower exterior member 5 and the upper exterior member 7 are unstretchable members, the exterior member itself does not contract, and therefore the exterior member deforms so that its surface creases on the first area and the second areas.

The lower exterior member 5 and the upper exterior member 7, which have been cut and separated from the lower bandlike member 15 and the upper bandlike member 17, are transported being absorbed on the anvil roll 51, and are ejected as a single piece of a diaper 1 to outside the welding-and-cutting apparatus 50. Through these processes, the diaper 1 is manufactured.

### < Modified Example >

In the foregoing embodiment, the adhesion areas HMA are formed by applying adhesive such as hot-melt type adhesive onto the first area, the first area being defined on the bandlike member which are being transported (S103 in FIG. 3). However, a method for forming the adhesion areas HMA is not limited thereto. For example, the adhesion areas HMA may be formed as follows: adhesive is applied to certain areas of the elastic members, these elastic members being stretched in the transport direction; and then the leg elastic members 8L and other elastic members are placed on at least either one of the lower bandlike member 15 and the upper bandlike member 17. That is, adhesion-area forming process (S103) and elastic-member placing process (S104) in FIG. 3 may be performed simultaneously. The following methods may be employed: applying adhesive to the elastic members and to at least either one of the lower bandlike member 15 and the upper bandlike member 17; or applying adhesive to only the elastic members.

Applying the adhesive to the elastic members increases adhesion of the elastic members on the adhesion areas HMA, and this makes it easier to prevent problems such as elastic drop-off. If the adhesion-area forming process and the elastic-member placing process are simultaneously performed, the diapers 1 can efficiently be manufactured. However, when applying adhesive only to the elastic members, it should be careful not to displace the positions of the adhesion areas HMA in the transport direction. That is, it is necessary to make adjustment so that the areas in which adhesive is applied to the elastic members and the positions where the elastic members are placed on the band-like substrate are not displaced.

When adhesive is applied onto an elastic member, an amount of adhesive applied is different between the following parts of the elastic member: the part which is placed within the first area; and the part which is placed within the second area. For example, adhesive is not applied onto the second areas. Or, the basis weight of adhesive applied to the part of the elastic member within the first area is larger at least than the basis weight of adhesive applied to the part of the elastic member within the second area. In other words, concerning adhesive which is applied in peripheral parts of the elastic member, the amount (weight) per unit area of the adhesive within the first area is larger than that of the adhesive within the second area. If adhesive is put in the foregoing manner, welding strength when welding is performed in the second areas may be larger than welding strength when welding is performed in the first area.

### === Second Embodiment ===

In the second embodiment, a method for manufacturing a disposable diaper consisting of three pieces (diaper 2) will be described.

### < Basic Configuration of Diaper 2 >

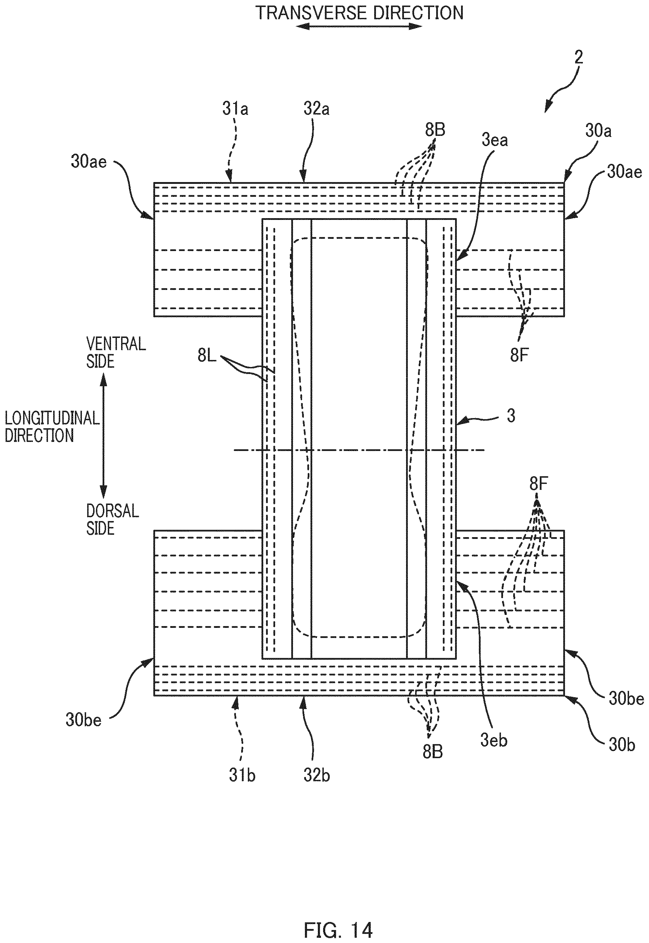

FIG. 14 is a plan view of a disposable diaper 2 which is spread out. In FIG. 14, like the first embodiment, the longitudinal direction and the transverse direction are defined. The thickness direction (not shown) which intersects the longitudinal direction and the transverse direction is also defined.

The diaper 2 which consists of three pieces and is manufactured in the second embodiment includes, as a first component, an absorbent main body 3 which is to be brought into contact with a wearer's crotch and to absorb excrement such as urine. The diaper 2 includes: a ventral band member 30a which covers a wearer's ventral part, as a second component; and a dorsal band member 30b which covers a wearer's dorsal part, as a third component. As for the diaper 2 in FIG. 14 which is spread out, the ventral band member 30a and the dorsal band member 30b are arranged at an interval in the longitudinal direction parallel to each other. In addition, while the absorbent main body 3 bridging between the members 30a and 30b, the lengthwise end sections 3ea and 3eb of the absorbent main body 3 are respectively joined and fixed to the nearest band members 30a and 30b. And, the appearance is in a substantially H shape when viewed from above. The absorbent main body 3 in this state is two-folded on the center in the lengthwise direction (the longitudinal direction). The ventral band member 30a and the dorsal band member 30b which faces each other with being two-folded are joined and connected on a ventral-band-member edge section 30ae and a dorsal-band-member edge section 30be which are to be in contact with wearer's sides (in other words, the both end sections in the transverse direction), and thereby these band members 30a and 30b are formed to be a ring. Thus, the diaper 2 which is worn is formed.

Both of the ventral band member 30a and the dorsal band member 30b are made of flexible sheets such as nonwoven fabric, and are a sheet member having a substantially rectangular shape when viewed from above. In the second embodiment, the ventral band member 30a is formed by stacking and joining a piece of lower nonwoven fabric 31a and a piece of upper nonwoven fabric 32a. And also, the dorsal band member 30b is formed by stacking and joining a piece of lower nonwoven fabric 31b and a piece of upper nonwoven fabric 32b. While being stretched along the transverse direction, the waist elastic members 8B and the fitting-gather elastic members 8F are placed between the lower nonwoven fabric 31a and 31b and the upper nonwoven fabric 32a and 32b. Stretchability is thereby provided to the ventral band member 30a and the dorsal band member 30b of the diaper 2.

On the transverse end sections of the absorbent main body 3, the leg-gather elastic members 8L are placed while being stretched along the longitudinal direction. And, stretchability is thereby provided to the absorbent main body 3 (stretchability is provided around the wearer's legs).

The detailed description of components of the diaper 2 is omitted because a disposable diaper consisting of three pieces itself is publicly known.

### < Method for Manufacturing Diaper 2 >

A method for manufacturing diapers 2 of the second embodiment will be described.

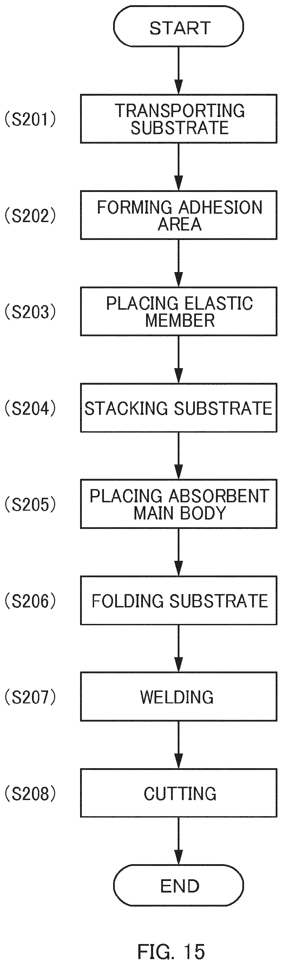

FIG. 15 is a flow chart of processes for manufacturing the diapers 2 in the second embodiment. the diapers 2 are continuously manufactured by performing processes (S201 to S208 in FIG. 15) in a manufacturing line. The processes S201 to S208 in the second embodiment are basically the same as the processes S101 to S108 in the first embodiment.

First, the substrate of the diapers 2 is transported along a certain transport direction at a certain transport speed (S201). The "substrate of the diapers 2" is composed of a ventral bandlike member 31al (a ventral bandlike member 32al) and a dorsal bandlike member 31bl (a dorsal bandlike member 32bl). The ventral bandlike member 31al (the ventral bandlike member 32al) is a member in which a plurality of pieces of the lower nonwoven fabric 31a (the upper nonwoven fabric 32a) are continuously linked in the transverse direction; the lower nonwoven fabric 31a (the upper nonwoven fabric 32a) constitutes the ventral band member 30a. Also, the dorsal bandlike member 31bl (the dorsal bandlike member 32bl) is a member in which a plurality of pieces of the lower nonwoven fabric 31b (the upper nonwoven fabric 32b) are continuously linked in the transverse direction; the lower nonwoven fabric 31b (the upper nonwoven fabric 32b) constitutes the dorsal band member 30b. These members are transported in the transport direction while the members each keep their position in relation to each other at certain distances in the intersecting direction. The definitions of "the transport direction" and "intersecting direction" are the same as in the first embodiment.

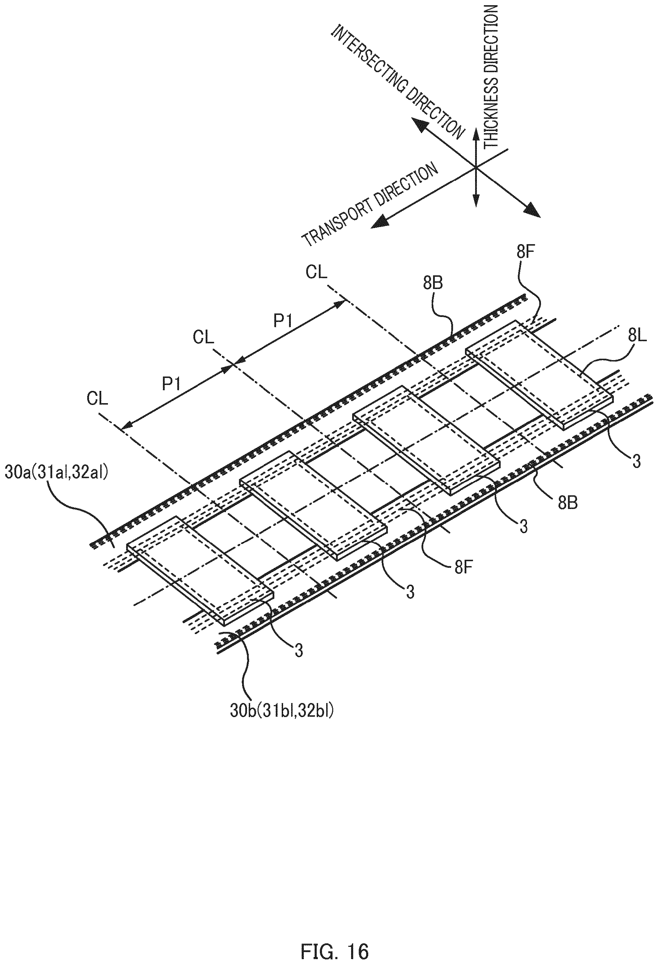

FIG. 16 is a diagram illustrating how the substrate of the diapers 2 is transported in the transport direction. FIG. 16 shows a state after the absorbent main body 3 bridges between the ventral band member 30a and the dorsal band member 30b in the process S205. In the second embodiment, as in the first embodiment, a cutting position CL is defined which is to be sections 30ae and 30be serving as the transverse end sections of a diaper 2 which is shaped in the form of underpants. Also, in the second embodiment, the operations of the processes are controlled based on the cutting position CL.

Hot-melt type adhesive is put on the first areas of the substrate which is being transported, and the adhesion areas HMA are formed (S202). The first areas where the adhesion areas HMA are formed are substantially the same as in the first embodiment. Each first area includes a cutting position CL on the substrate, and has a certain width in the transport direction. Thereafter, the waist elastic members 8B and the fitting-gather elastic members 8F are placed while being stretched in the transport direction (S203). At this stage, the elastic members 8B and 8F are placed so that the partial areas of the elastic members 8B and 8F respectively overlap the adhesion areas HMA formed in the first area. And, the elastic members 8B and 8F adhere to the ventral bandlike member 31al and the dorsal bandlike member 31bl on the overlapping area. After the elastic members are placed, the ventral bandlike member 32al is stacked on the ventral bandlike member 31al and the elastic members 8B and 8F (S204). Accordingly, the elastic members 8B and 8F are fixed to the substrates (the ventral bandlike member 31al and the ventral bandlike member 32al) while being sandwiched in the thickness direction between the substrates. The elastic members 8B and 8F can exert stretching-and-contraction force in the transverse direction (transport direction) on the edge sections 30ae and 30be of the diaper 2. Similarly, the dorsal bandlike member 32bl is stacked on the dorsal bandlike member 31bl and the elastic members 8B and 8F. In the foregoing processes, the lower nonwoven fabric may be stacked on the upper nonwoven fabric which is being transported.

Subsequently, the absorbent main body 3 is placed and joined so as to bridge between the ventral band member 30a and the dorsal band member 30b (S205). With hot-melt adhesive, etc., the ventral band member 30a and the dorsal band member 30b is joined to the absorbent main body 3. FIG. 16 shows a state after the absorbent main body 3 has been joined in this process. The absorbent main body 3 is folded on the center in the intersecting direction so that the ventral band member 30a and the dorsal band member 30b are stacked in the thickness direction (S206).

Thereafter, the ventral band member 30a and the dorsal band member 30b which are stacked are welded on the second areas (S207). The second areas are substantially the same as in the first embodiment. The second areas are respectively place on both sides of the first area in the transport direction, and each have a certain width in the transport direction. The operation of the welding process itself is substantially the same as in the first embodiment, and is performed using the welding-and-cutting apparatus 50. Thus, a plurality of the welded sections sl are formed at intervals along the intersecting direction so that some of the welded sections partially overlap the adhesion areas HMA. In the second embodiment, welded sections sl in which a small amount per unit area of adhesive is applied (or adhesive is not applied) within the second area are formed, and thereby, welding can be performed so that sufficient welding strength is ensured.

Finally, the ventral band member 30a, the dorsal band member 30b and the elastic members 8B and 8F are cut together at the cutting position CL (S208). The operation of cutting process is also substantially the same as in the first embodiment, and the elastic members 8B and 8F which are stretched in the transport direction contract along the transport direction towards a direction away from the cutting position CL. As the elastic members contract, the band member edge sections 30ae and 30be deform so as to shrink along the transport direction. Since the edge sections of the diaper 2 which extend protruding transversely outwardly shrink transversely inwardly, the edge sections become less likely to be noticeable. This makes the appearance of the diaper 2 good.

### === Other Embodiments ===

While the embodiments according to the invention are described above, the above-mentioned embodiments are provided for facilitating the understanding of the invention, and are not to be interpreted as limiting the invention. As a matter of course, the invention can be altered and improved without departing from the gist thereof and the invention includes equivalent thereof. For example, the invention can be altered as described below.

In the foregoing embodiment, after the elastic members are placed on the lower substrate (e.g., the lower bandlike member 15) which is being transported, the exterior member of an absorbent article is formed by stacking on and joining to the upper substrate (e.g., the upper bandlike member 17). However, a process for forming the exterior member is not limited thereto. For example, the elastic members may be placed between the lower substrate and the upper substrate which are being transported in parallel in a state in which these substrates faces each other. In any way, a method for manufacturing an absorbent article according to the present application can apply to any configuration for forming an exterior member in which the elastic members are placed, the elastic members being stretched and sandwiched between a first substrate and a second substrate.

In the foregoing embodiment, using the welding-and-cutting apparatus 50, the welding process (S106, for example) and the cutting process (S107) are performed as a series of operations. However, these processes may be performed individually. For example, the following configuration is acceptable. Using a first anvil roll and a second anvil roll instead of the anvil roll 51 of the welding-and-cutting apparatus 50, the welding process is performed while the band-like substrate is being transported by the first anvil roll. And then, the cutting process is performed while the band-like substrate is being transported by the second anvil roll. In addition, a configuration in which the welding process alone is performed with the anvil roll and the cutting process is individually performed with another cutting device may be employed. Further, any other configuration may be employed depending on the place or conditions of a manufacturing line.

In the foregoing embodiment, nonwoven fabric is provided as an example of the materials of the lower exterior member 5 and the upper exterior member 7. However, the materials are not limited to nonwoven fabric. For example, woven fabric or any other sheet member except for woven fabric may also be used.

In the foregoing embodiment, rubber thread is provided as an example of the elastic members 8. However, this invention is not limited thereto. For example, band-like rubber may be used as the elastic members 8, and band-like nonwoven fabric with stretchability or band-like resin film with stretchability may also be used.

### Reference Signs List

1 diaper (absorbent article),
1hB waist opening, 1hL leg opening,
2 diaper (absorbent article)
3 absorbent main body, 3b back face sheet, 3d absorbent body, 3s top face sheet,
3ea end section, 3eb end section,
5 lower exterior member,
7 upper exterior member,
8B waist elastic member, 8F fitting-gather elastic member, 8L leg elastic member,
10 exterior member,
10A ventral part, 10ae transverse direction end section,
10B crotch part,
10C dorsal part, 10ce transverse direction end section,
10e edge section, 10eL edge section, 10s transverse direction end section,
15 lower bandlike member,
17 upper bandlike member,
30a ventral band member,, 30ae ventral-band-member edge section,
30b dorsal band member, 30be dorsal-band-member edge section,
31a lower nonwoven fabric, 31al ventral bandlike member,
31b lower nonwoven fabric, 31bl dorsal bandlike member,
32a upper nonwoven fabric, 32al ventral bandlike member,
32b upper nonwoven fabric, 32bl dorsal bandlike member,
50 welding-and-cutting apparatus,
51 anvil roll, 511 anvil brock,
52 sealing roll, 521 heating block, 522 protruding section, 522a rectangular portion,
53 cutter roll, 531 cutter block,
54 transportation-assisting roll,
CL cutting position,
HMA adhesion area,
sl welded section

## Claims

1. A method for continuously manufacturing an absorbent article,
the absorbent article (2) being a pull-on absorbent article
that has a longitudinal direction and a transverse direction intersecting the longitudinal direction, and
that includes
a lower exterior member (5) located on a side towards a wearer's garment,
an upper exterior member (7) stacked on the lower exterior member from a side towards a wearer's skin,
an absorbent main body (3) stacked on the upper exterior member (7) from the side towards a wearer's skin, for absorbing excrement,
the method, comprising:
while a lower bandlike member (15) and an upper bandlike member (17) are transported in a transport direction that is along the transverse direction,
the lower bandlike member (15) including the lower exterior members (5) that continue in a band-like manner in the transverse direction,
the upper bandlike member (17) including the upper exterior members (7) that continue in a band-like manner in the transverse direction,
a process in which,
a cutting position (CL) is determined, and
an adhesion area (HMA) is formed by putting adhesive on a first area of at least either one of the lower bandlike member (15) and the upper bandlike member (17),
the cutting position being to serve as end sections of the lower exterior member (5) and the upper exterior member (7) in the transverse direction when the lower exterior member (5) and the upper exterior member (7) are cut and separated from the lower bandlike member (15) and the upper bandlike member (17),
the first area including the cutting position (CL) and having a predetermined width in the transport direction;
a process in which
a plurality of elastic members (8B, 8F, 8L) are placed so that a part of each of the elastic members (8B, 8F, 8L) overlaps the adhesion area,
the elastic members (8B, 8F, 8L) being stretched in the transport direction;
a process in which
the lower bandlike member (15) and the upper bandlike member (17) are stacked such that the elastic members are sandwiched between the lower and upper bandlike members (15, 17), and
the upper bandlike member (17) adheres to the lower bandlike member (15) and the elastic members (8B, 8F, 8L) on the adhesion area (HMA);
a process in which
the lower bandlike member (15) and the upper bandlike member (17) are folded on a center portion in an intersecting direction so that the upper bandlike member (17) is located inside the lower bandlike member (15),
the intersecting direction intersecting the transport direction;
a process in which
a welded section (s1) on which the folded lower bandlike member (15) and the folded upper bandlike member (17) are to be welded to each other is formed on a second area,
the second area being located on both sides of the first area in the transport direction and having a predetermined width in the transport direction; and
a process in which
the lower bandlike member (15), the upper bandlike member (17) and the elastic members (8B, 8F, 8L) are cut together on the cutting position (CL), and
the lower exterior member (5) and the upper exterior member (7) which are shaped in the form of underpants are cut and separated from the lower bandlike member (15) and the upper bandlike member (17);
wherein
the second area does not overlap the first area within at least a partial area along the transport direction,
the second area is defined so that a distance (xsl) from the cutting position (CL) to an end section of the second area in the transport direction that is closer to the cutting position is smaller than a distance (x) from the cutting position to an end section of the first area in the transport direction.

2. A method for manufacturing an absorbent article according to claim 1, wherein
the adhesive is not put on the second area.

3. A method for manufacturing an absorbent article according to claim 1, wherein
the adhesive is put on the second area, and
an amount per unit area of the adhesive that has been put on the second area is smaller than an amount per unit area of the adhesive that has been put on the first area.

4. A method for manufacturing an absorbent article according to claim 1, wherein
the adhesive is put on the second area, and
an amount per unit area of the adhesive which is put on the first area and the second area is determined so that
welding strength in a case in which the lower bandlike member (15) and the upper bandlike member (17) are welded in the second area on which the adhesive is put
is larger than
welding strength in a case in which the lower bandlike member (15) and the upper bandlike member (17) are welded in the first area on which the adhesive is put.

5. A method for manufacturing an absorbent article according to any one of claims 1 to 4, wherein
a plurality of the welded sections (s1) are formed at intervals along the intersecting direction, and
a distance between two welded sections that are formed adjacent in the intersecting direction is larger than a diameter of the elastic member.

6. A method for manufacturing an absorbent article according to any one of claims 1 to 4, wherein
the process in which the adhesion area (HMA) is formed is performed by placing the elastic members on at least either one of the lower bandlike member (15) and the upper bandlike member (17),
adhesive being applied to the elastic members on a certain area while the elastic members being stretched in the transport direction.

## Patentansprüche

1. Verfahren für die kontinuierliche Herstellung eines absorbierenden Artikels, wo
bei der absorbierende Artikel (2) ein absorbierender Schlupfartikel ist,
der eine Längsrichtung und eine Querrichtung, die die Längsrichtung überkreuzt, aufweist, und
der Folgendes einschließt:
ein unteres äußeres Element (5), das sich an einer Seite in Richtung der Kleidung eines Trägers befindet,
ein oberes äußeres Element (7), das auf das untere äußere Element von einer Seite in Richtung der Haut eines Trägers geschichtet ist,
einen absorbierenden Hauptkörper (3), der auf das obere äußere Element (7) von der Seite in Richtung der Haut eines Trägers geschichtet ist, für die Absorbierung von Exkrementen,
wobei das Verfahren Folgendes umfasst:
während ein unteres bandartiges Element (15) und ein oberes bandartiges Element (17) in einer Transportrichtung transportiert werden, die entlang der Querrichtung verläuft,
wobei das untere bandartige Element (15) die unteren äußeren Elemente (5) einschließt, die in einer bandartigen Weise in der Querrichtung fortlaufen,
wobei das obere bandartige Element (17) die oberen äußeren Elemente (7) einschließt, die in einer bandartigen Weise in der Querrichtung fortlaufen,
einen Vorgang, in dem
eine Schneideposition (CL) bestimmt wird und
eine Haftfläche (HMA) durch Auftragen von Haftmittel auf eine erste Fläche von mindestens einem des unteren bandartigen Elements (15) und des oberen bandartigen Elements (17) gebildet wird,
wobei die Schneideposition als Endabschnitte des unteren äußeren Elements (5) und des oberen äußeren Elements (7) in der Querrichtung dient, wenn das untere äußere Element (5) und das obere äußere Element (7) geschnitten und von dem unteren bandartigen Element (15) und dem oberen bandartigen Element (17) getrennt werden,
wobei die erste Fläche die Schneideposition (CL) einschließt und eine vorgegebene Breite in der Transportrichtung aufweist;
einen Vorgang, in dem
eine Vielzahl von elastischen Elementen (8B, 8F, 8L) derart platziert wird, dass ein Teil von jedem der elastischen Elemente (8B, 8F, 8L) mit der Haftfläche überlappt,
wobei die elastischen Elemente (8B, 8F, 8L) in der Transportrichtung gedehnt werden;
einen Vorgang, in dem
das untere bandartige Element (15) und das obere bandartige Element (17) geschichtet werden, sodass die elastischen Elemente zwischen dem unteren und oberen bandartigen Element (15, 17) eingeschoben sind, und
das obere bandartige Element (17) auf dem unteren bandartigen Element (15) und den elastischen Elementen (8B, 8F, 8L) auf der Haftfläche (HMA) haftet;
einen Vorgang, in dem
das untere bandartige Element (15) und das obere bandartige Element (17) an einem mittleren Abschnitt in einer überkreuzenden Richtung derart gefaltet werden, dass sich das obere bandartige Element (17) innerhalb des unteren bandartigen Elements (15) befindet,
wobei sich die überkreuzende Richtung mit der Transportrichtung überkreuzt;
einen Vorgang, in dem
ein verschweißter Abschnitt (sl), auf dem das gefaltete untere bandartige Element (15) und das gefaltete obere bandartige Element (17) miteinander zu verschweißen sind, auf einer zweiten Fläche gebildet wird,
wobei sich die zweite Fläche auf beiden Seiten der ersten Fläche in der Transportrichtung befindet und eine vorgegebene Breite in der Transportrichtung aufweist; und
einen Vorgang, in dem
das untere bandartige Element (15), das obere bandartige Element (17) und die elastischen Elemente (8B, 8F, 8L) zusammen an der Schneideposition (CL) geschnitten werden und
das untere äußere Element (5) und das obere äußere Element (7), die in der Form von Unterhosen geformt sind, geschnitten und von dem unteren bandartigen Element (15) und dem oberen bandartigen Element (17) getrennt werden;
wobei
die zweite Fläche nicht mit der ersten Fläche innerhalb von mindestens einer Teilfläche entlang der Transportrichtung überlappt,
wobei die zweite Fläche derart definiert ist, dass eine Entfernung (xsl) von der Schneideposition (CL) zu einem Endabschnitt der zweiten Fläche in der Transportrichtung, der näher zu der Schneideposition ist, kleiner ist als eine Entfernung (x) von der Schneideposition zu einem Endabschnitt der ersten Fläche in der Transportrichtung.

2. Verfahren für die Herstellung eines absorbierenden Artikels nach Anspruch 1, wobei
das Haftmittel nicht auf die zweite Fläche aufgetragen wird.

3. Verfahren für die Herstellung eines absorbierenden Artikels nach Anspruch 1, wobei
das Haftmittel auf die zweite Fläche aufgetragen wird und
eine Menge pro Flächeneinheit des Haftmittels, das auf die zweite Fläche aufgetragen wurde, kleiner ist als eine Menge pro Flächeneinheit des Haftmittels, das auf die erste Fläche aufgetragen wurde.

4. Verfahren für die Herstellung eines absorbierenden Artikels nach Anspruch 1, wobei
das Haftmittel auf die zweite Fläche aufgetragen wird und
eine Menge pro Flächeneinheit des Haftmittels, das auf die erste Fläche und die zweite Fläche aufgetragen wird, derart bestimmt wird, dass
die Schweißfestigkeit in einem Fall, in dem das untere bandartige Element (15) und das obere bandartige Element (17) in der zweiten Fläche, auf die das Haftmittel aufgetragen wird, verschweißt werden,
größer ist als
die Schweiß festigkeit in einem Fall, in dem das untere bandartige Element (15) und das obere bandartige Element (17) in der ersten Fläche, auf die das Haftmittel aufgetragen wird, verschweißt werden.

5. Verfahren für die Herstellung eines absorbierenden Artikels nach einem der Ansprüche 1 bis 4, wobei
eine Vielzahl der verschweißten Abschnitte (s1) in Abständen entlang der überkreuzenden Richtung ausgebildet wird und
eine Entfernung zwischen zwei verschweißten Abschnitten, die benachbart in der überkreuzenden Richtung ausgebildet sind, größer ist als ein Durchmesser des elastischen Elements.

6. Verfahren für die Herstellung eines absorbierenden Artikels nach einem der Ansprüche 1 bis 4, wobei
der Vorgang, in dem die Haftfläche (HMA) gebildet wird, durch Platzieren der elastischen Elemente auf mindestens eines des unteren bandartigen Elements (15) und des oberen bandartigen Elements (17) durchgeführt wird,
wobei das Haftmittel auf die elastischen Elemente auf einer bestimmten Fläche aufgetragen wird, während die elastischen Elemente in der Transportrichtung gedehnt werden.

## Revendications

1. Procédé de fabrication en continu d'un article absorbant, l'article absorbant (2) étant un article absorbant du type à enfiler
qui a une direction allant dans le sens longitudinal et une direction allant dans le sens transversal croisant la direction allant dans le sens longitudinal, et
qui comprend
un élément extérieur inférieur (5) se trouvant sur un côté orienté vers un vêtement d'un utilisateur,
un élément extérieur supérieur (7) empilé sur l'élément extérieur inférieur depuis un côté orienté vers la peau d'un utilisateur,
un corps principal absorbant (3) empilé sur l'élément extérieur supérieur (7) depuis un côté orienté vers la peau d'un utilisateur, à des fins d'absorption d'excrément,
le procédé, comportant :
alors qu'un élément inférieur similaire à une bande (15) et qu'un élément supérieur similaire à une bande (17) sont transportés dans une direction allant dans le sens du transport qui se trouve le long de la direction allant dans le sens transversal,
l'élément inférieur similaire à une bande (15) comprenant les éléments extérieurs inférieurs (5) qui continuent d'une manière similaire à une bande dans la direction allant dans le sens transversal,
l'élément supérieur similaire à une bande (17) comprenant les éléments extérieurs supérieurs (7) qui continuent d'une manière similaire à une bande dans la direction allant dans le sens transversal,
un processus dans lequel,
une position de découpe (CL) est déterminée, et
une zone d'adhésion (HMA) est formée en mettant de l'adhésif sur une première zone d'au moins l'un quelconque parmi l'élément inférieur similaire à une bande (15) et l'élément supérieur similaire à une bande (17),
la position de découpe ayant pour objet de servir de sections d'extrémité de l'élément extérieur inférieur (5) et de l'élément extérieur supérieur (7) dans la direction allant dans le sens transversal quand l'élément extérieur inférieur (5) et l'élément extérieur supérieur (7) sont découpés et séparés de l'élément inférieur similaire à une bande (15) et de l'élément supérieur similaire à une bande (17),
la première zone comprenant la position de découpe (CL) et ayant une largeur prédéterminée dans la direction allant dans le sens du transport ;
un processus dans lequel
une pluralité d'éléments élastiques (8B, 8F, 8L) sont placés de telle sorte qu'une partie de chacun des éléments élastiques (8B, 8F, 8L) chevauche la zone d'adhésion,
les éléments élastiques (8B, 8F, 8L) étant étirés dans la direction allant dans le sens du transport ;
un processus dans lequel
l'élément inférieur similaire à une bande (15) et l'élément supérieur similaire à une bande (17) sont empilés de telle sorte que les éléments élastiques sont pris en sandwich entre les éléments inférieur et supérieur similaires à une bande (15, 17), et
l'élément supérieur similaire à une bande (17) adhère au niveau de l'élément inférieur similaire à une bande (15) et au niveau des éléments élastiques (8B, 8F, 8L) sur la zone d'adhésion (HMA) ;
un processus dans lequel
l'élément inférieur similaire à une bande (15) et l'élément supérieur similaire à une bande (17) sont pliés sur une partie centrale dans une direction allant dans le sens du croisement de telle sorte que l'élément supérieur similaire à une bande (17) est situé à l'intérieur de l'élément inférieur similaire à une bande (15),
la direction allant dans le sens du croisement croisant la direction allant dans le sens du transport ;
un processus dans lequel
une section soudée (s1), sur laquelle l'élément inférieur similaire à une bande plié (15) et l'élément supérieur similaire à une bande plié (17) doivent être soudés l'un par rapport à l'autre, est formée sur une deuxième zone,
la deuxième zone étant située des deux côtés de la première zone dans la direction allant dans le sens du transport et ayant une largeur prédéterminée dans la direction allant dans le sens du transport ; et
un processus dans lequel
l'élément inférieur similaire à une bande (15), l'élément supérieur similaire à une bande (17) et les éléments élastiques (8B, 8F, 8L) sont découpés ensemble sur la position de découpe (CL), et
l'élément extérieur inférieur (5) et l'élément extérieur supérieur (7) qui sont façonnés sous la forme d'une culotte sont découpés et séparés de l'élément inférieur similaire à une bande (15) et de l'élément supérieur similaire à une bande (17) ;
dans lequel
la deuxième zone ne chevauche pas la première zone dans les limites d'au moins une zone partielle le long de la direction allant dans le sens du transport,
la deuxième zone est définie de telle sorte qu'une distance (xsl) depuis la position de découpe (CL) jusqu'à une section d'extrémité de la deuxième zone dans la direction allant dans le sens du transport qui est plus proche de la position de découpe est inférieure par rapport à une distance (x) depuis la position de découpe jusqu'à une section d'extrémité de la première zone dans la direction allant dans le sens du transport.

2. Procédé de fabrication d'un article absorbant selon la revendication 1, dans lequel l'adhésif n'est pas placé sur la deuxième zone.

3. Procédé de fabrication d'un article absorbant selon la revendication 1, dans lequel
l'adhésif est placé sur la deuxième zone, et
une quantité par unité de surface de l'adhésif qui a été placé sur la deuxième zone est inférieure par rapport à une quantité par unité de surface de l'adhésif qui a été placé sur la première zone.

4. Procédé de fabrication d'un article absorbant selon la revendication 1, dans lequel
l'adhésif est placé sur la deuxième zone, et
une quantité par unité de surface de l'adhésif qui est placé sur la première zone et sur la deuxième zone est déterminée de telle sorte que
la résistance du soudage dans un cas de figure dans lequel l'élément inférieur similaire à une bande (15) et l'élément supérieur similaire à une bande (17) sont soudés dans la deuxième zone sur laquelle l'adhésif est placé
est supérieure par rapport à
la résistance du soudage dans un cas de figure dans lequel l'élément inférieur similaire à une bande (15) et l'élément supérieur similaire à une bande (17) sont soudés dans la première zone sur laquelle l'adhésif est placé.

5. Procédé de fabrication d'un article absorbant selon l'une quelconque des revendications 1 à 4, dans lequel
une pluralité de sections soudées (s1) sont formées selon des intervalles le long de la direction allant dans le sens du croisement, et
une distance entre deux sections soudées qui sont formées de manière adjacente dans la direction allant dans le sens du croisement est supérieure par rapport à un diamètre de l'élément élastique.

6. Procédé de fabrication d'un article absorbant selon l'une quelconque des revendications 1 à 4, dans lequel
le processus dans lequel la zone d'adhésion (HMA) est formée est effectué en plaçant les éléments élastiques sur au moins l'un ou l'autre parmi l'élément inférieur similaire à une bande (15) et l'élément supérieur similaire à une bande (17),
de l'adhésif étant appliqué sur les éléments élastiques sur une certaine zone alors que les éléments élastiques sont étirés dans la direction allant dans le sens du transport.
